# EUROPÄISCHE PATENTSCHRIFT

(11) **EP 2 992 081 B1**
(45) Veröffentlichungstag und Bekanntmachung des Hinweises auf die Patenterteilung: **06.05.2020**
(21) Anmeldenummer: 14714936.3
(22) Anmeldetag: 22.03.2014
(51) Int. Cl.: C12M 1/12, C12M 1/36

(54) **SYSTEM ZUR ABLUFTUMSCHALTUNG EINES BIOREAKTORS**
SYSTEM FOR SWITCHING OVER THE EXHAUST OF A BIOREACTOR
SYSTÈME POUR LA COMMUTATION DE L'AIR D'ÉCHAPPEMENT D'UN BIORÉACTEUR

(30) Priorität: 03.05.2013 DE 202013004096 U
(43) Veröffentlichungstag der Anmeldung: 09.03.2016
(73) Patentinhaber: Sartorius Stedim Biotech GmbH, 37079 Göttingen (DE)
(72) Erfinder: KAHLERT, Wolfgang, 34327 Körle (DE); HARTMANN, Ralf, 37308 Döringsdorf (DE); HUSEMANN, Bernward, 37079 Göttingen (DE)
(74) Vertreter: Stehl, Astrid
(86) Internationale Anmeldenummer: PCT/EP2014/000782
(87) Internationale Veröffentlichungsnummer: WO 2014/177240

(56) Entgegenhaltungen:
- WO-A1-2010/046029
- WO-A1-2011/041508
- WO-A1-2012/049290
- WO-A1-2013/053779
- WO-A2-2008/135991
- DE-A1- 4 236 856
- DE-A1-102006 004 157
- DE-A1-102008 033 286
- GB-A- 191 026 773
- US-A1- 2011 207 170

## Beschreibung

### Gebiet der Erfindung

Die Erfindung betrifft ein System zur Abluftumschaltung eines Bioreaktors, dessen Einwegbehälter über mindestens zwei Abluftleitungen mit Abluftfiltern in Verbindung steht.

### Stand der Technik

Bioreaktoren mit Abluftleitungen weisen üblicherweise zum Schutz vor Kontamination nach innen und nach außen in ihrer Abluftleitung einen Abluftfilter auf. Im Gebrauch können diese Abluftfilter verblocken, was zu einem unerwünschten Druckanstieg im Bioreaktor führt. Um beispielsweise ein unerwünschtes Platzen eines Einwegbehälters zu verhindern, muss der Prozess unterbrochen und der Abluftfilter ausgetauscht werden.

Aus der US 2009/0269849 A1 ist ein Bioreaktorsystem bekannt, dessen beutelförmiger Behälter über zwei Abluftleitungen mit Abluftfiltern in Verbindung steht. Über die Abluftleitungen mit den Abluftfiltern kann dabei ein Druckausgleich erfolgen.

Nachteilig dabei ist, dass die Verwendung von zwei Abluftleitungen mit zwei Abluftfiltern zwar die Standzeit bis zur Verblockung erhöht, eine Verblockung aber nicht verhindern kann. Bei einer Verblockung muss auch hier der Prozess unterbrochen werden.

Weiterhin ist aus der US 2011/0207218 A1 ein Bioreaktorsystem bekannt, welches einen beutelförmigen Bioreaktor umfasst, bei dem eine Gasabführungsleitung über einen beutelförmigen Abluftkühler mit zwei von dem Abluftkühler abzweigenden Abluftleitungen verbunden ist. Die Abluftleitungen weisen ihrerseits jeweils einen Sterilfilter auf. Nachteilig dabei ist, dass eine Verblockung eines Filters sowohl durch feuchte Abluft als auch durch Partikel verursacht werden kann. Aus diesem Grund kann nicht immer ein Abluftkühler eine Verblockung verhindern und ein Wechsel der Filter kann notwendig werden. Auch hier muss dann der Prozess unterbrochen werden.

WO 2008/135991 A2 offenbart einen Labor-Einweg-Bioreaktor mit mehreren Gaseinlassleitungen am Boden des Behälters und mit mehreren Gasabluftleitungen mit Zugängen an der Oberseite des Bioreaktors. Die Gaseinlassleitungen sind mit Filtern versehen, wie auch die Abluftleitungen.

### Aufgabenstellung

Aufgabe der vorliegenden Erfindung ist es daher, einen Druckanstieg im Einwegbehälter infolge Verblockung der Abluftfilter zu verhindern und eine Prozessunterbrechung zu vermeiden.

### Darlegung der Erfindung

Die Aufgabe wird mit den Merkmalen des Anspruches 1 dadurch gelöst, dass in mindestens einer der mindestens zwei Abluftleitungen ein Ventil angeordnet ist, über das der zugehörige Abluftfilter freischaltbar ist.

Durch das dem Abluftfilter vor- oder nachgeschaltete Ventil wird sichergestellt, dass der dem Ventil vor- oder nachgeschaltete Abluftfilter zum Zeitpunkt der Ventilöffnung unverblockt ist. Bei Erreichen eines bestimmten Druckes infolge Verblockung des ersten Abluftfilters wird das Ventil geöffnet und zu dem zweiten Abluftfilter umgeschaltet.

Die Abluftleitungen können dabei separat mit dem Einwegbehälter verbunden sein. Es ist aber auch möglich, dass die Abluftleitungen über ein Verteilerstück mit dem Einwegbehälter verbunden sind. Um die Zahl der Abluftfilter zu erhöhen, kann neben einer Mehrzahl von separaten Abluftleitungen und Abluftleitungen, die über ein Verteilerstück mit dem Einwegbehälter verbunden sind, auch eine Kombination von unterschiedlichen. Abluftleitungen angeordnet sein.

Nach einer weiteren bevorzugten Ausführungsform der Erfindung steht mindestens eine weitere Abluftleitung mit dem Einwegbehälter in Verbindung, die an ihrem freien Ende von einem Teil eines zweiteiligen Sterilverbinders verschlossen ist. Bei Bedarf können dann über den Sterilverbinder ein weiterer Abluftfilter und ein Ventil angeschlossen werden. Gemäß der Erfindung sind in den Abluftleitungen zum Einwegbehälter Drucksensoren vorgesehen. Bei einer weiteren Ausführungsform der Erfindung können die Drucksensoren in den Abluft- und in den Zuluftleitungen zum Einwegbehälter vorgesehen sein. Über die Drucksensoren kann insbesondere der Druck im Einwegbehälter ermittelt werden.

Gemäß der Erfindung ist eine Regel- und Steuereinheit vorgesehen. Die Regel- und Steuereinheit ist über Steuerleitungen mit den Ventilen verbunden. Über Sensorleitungen ist die Regel- und Steuereinheit mit den Drucksensoren verbunden. Durch die Regel- und Steuereinheit kann bei Erreichen eines vorgegebenen Grenzdruckes eine Abluftumschaltung automatisch erfolgen, in dem ein weiterer Abluftfilter durch Öffnen seines ihm zugeordneten Ventils zugeschaltet wird. Auch wenn die Ventile bevorzugt den Abluftfiltern vorgeschaltet angeordnet sind, ist es doch auch möglich, die Ventile den Abluftfiltern nachgeschaltet anzuordnen.

Gemäß einer weiteren bevorzugten Ausführungsform der Erfindung ist zwischen Einwegbehälter und Abluftfilter ein Abluftkühler angeordnet. Durch einen Abluftkühler ist es möglich, die Verblockung von Abluftfiltern infolge von Feuchtigkeit zu verringern. Weitere Merkmale der Erfindung ergeben sich aus der nachfolgenden ausführlichen Beschreibung und den beigefügten Zeichnungen, in denen bevorzugte Ausführungsformen der Erfindung beispielhaft veranschaulicht sind.

Kurzbeschreibung der Zeichnungen
- Figur 1:: eine prinzipielle Seitenansicht eines Systems zur Abluftumschaltung eines Bioreaktors mit separaten Abluftleitungen mit Abluftfiltern und einer Regel- und Steuereinheit;
- Figur 2:: ein weiteres Abluftsystem entsprechend Fig. 1 mit zusätzlichen Sensoren in den Abluftleitungen und in einer Zuluftleitung zum Einwegbehälter;
- Figur 3:: ein weiteres Abluftsystem entsprechend Fig. 1 mit einem zusätzlichen Ventil in der ersten Abluftleitung und nicht dargestellter Regel- und Steuereinheit;
- Figur 4;: ein weiteres Abluftsystem entsprechend Fig. 3 jedoch mit den Abluftfiltern in Abluftrichtung nachgelagerten Ventilen;
- Figur 5:: ein weiteres System zur Abluftumschaltung mit Abluftleitungen, die über ein Verteilerstück mit dem Einwegbehälter verbunden sind, wobei in dem Verteilerstück ein Ventil angeordnet ist;
- Figur 6:: ein weiteres System zur Abluftschaltung, bei dem die Abluftleitungen über ein Verteilerstück mit dem Einwegbehälter verbunden ist, wobei dem Verteilerstück zum Einwegbehälter hin ein Abluftkühler angeordnet ist;
- Figur 7:: ein Abluftsystem eines Bioreaktors zum Prozessstart ähnlich Fig. 1 mit einer zusätzlichen Abluftleitung, die von einem ersten Teil eines zweiteiligen Sterilverbinders verschlossen ist;
- Figur 8:: das System zur Abluftumschaltung von Fig. 7 mit verblocktem ersten Filter und geöffnetem, dem zweiten Filter vorgelagerten Ventil;
- Figur 9:: das System zur Abluftumschaltung von Fig. 8 mit auf den ersten Teil des zweiteiligen Sterilverbinders aufgesetztem zweiten Teil mit geschlossenem Ventil und nachgelagertem weiteren Abluftfilter;
- Figur 10:: den Prozessstart eines Systems zur Abluftumschaltung entsprechend Fig. 1 mit geschlossenem Ventil;
- Figur 11:: das System von Fig. 10 mit verblocktem ersten Filter und geöffnetem, dem zweiten Filter vorgelagerten Ventil;
- Figur 12:: das System von Fig. 11 mit verschweißter und abgetrennter erster Abluftleitung; und
- Figur 13:: das System zur Abluftumschaltung von Fig. 12 mit angeschweißtem neuen Schlauchstück mit Filter und verschlossenem Ventil.

### Beschreibung bevorzugter Ausführungsformen

Ein System 1 zur Abluftumschaltung eines Bioreaktors 2 besteht im Wesentlichen aus dessen Einwegbehälter 3, Abluftleitungen 4, 5 und Abluftfiltern 6, 7.

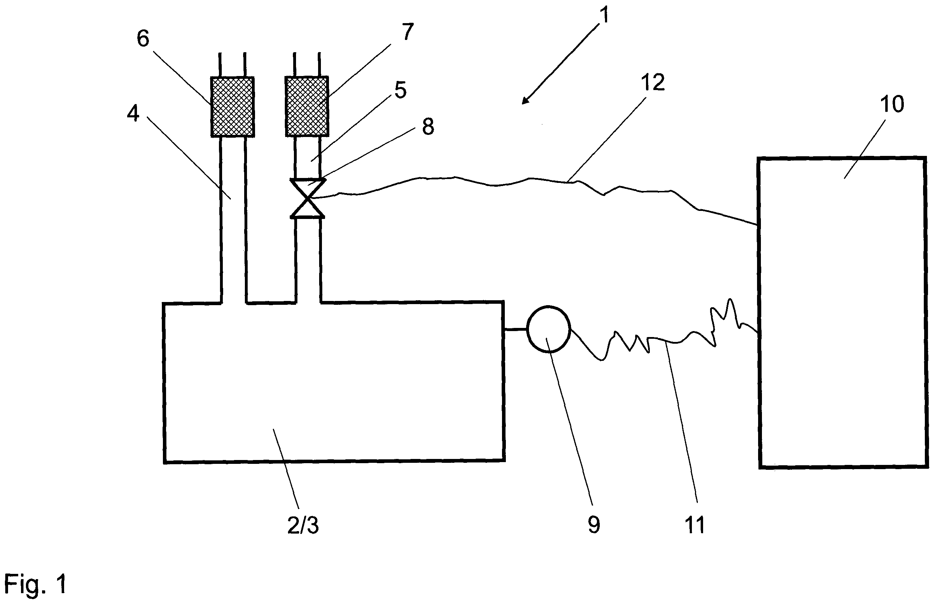

Entsprechend den Ausführungsbeispielen der Figuren 1 bis 4 und 7 bis 13 sind die Abluftleitungen 4, 5 separat mit dem Einwegbehälter 3 verbunden. Entsprechend dem Ausführungsbeispiel von Figur 1 ist in der zweiten Abluftleitung 5 zwischen dem Einwegbehälter 3 und zweitem Abluftfilter 7 ein Ventil 8 angeordnet. Das Ventil 8 kann beispielsweise als Quetschventil ausgebildet sein. Die Verwendung eines Quetschventils hat den Vorteil, dass es von außen auf eine als flexibler Schlauch ausgebildete Abluftleitung 5 aufsetzbar ist und nach dem Verblocken des Filters an anderer Stelle eingesetzt werden kann. Es ist aber auch grundsätzlich möglich, die Ventile 8 als Einwegventile (single-use-Ventil) auszubilden. Entsprechend dem Ausführungsbeispiel von Figur 1 steht der Innenraum des Einwegbehälters 3 mit einem Sensor 9, der als Drucksensor ausgebildet ist, in Verbindung.

Eine Regel- und Steuereinheit 10 steht über eine Sensorleitung 11 mit dem Drucksensor 9 in Verbindung. Über eine Steuerleitung 12 steht die Regel- und Steuereinheit 10 mit dem Ventil 8 in Verbindung. Über die Regel- und Steuereinheit 10 wird in Abhängigkeit von dem Druck im Einwegbehälter 3 das Ventil 8 in der zweiten Abluftleitung 5 geöffnet, so dass bei Druckanstieg infolge Verblockung des ersten Abluftfilters 6 der zweite Abluftfilter 7 freigegeben wird und somit die Abluft auf den zweiten Filter 7 umgeschaltet wird.

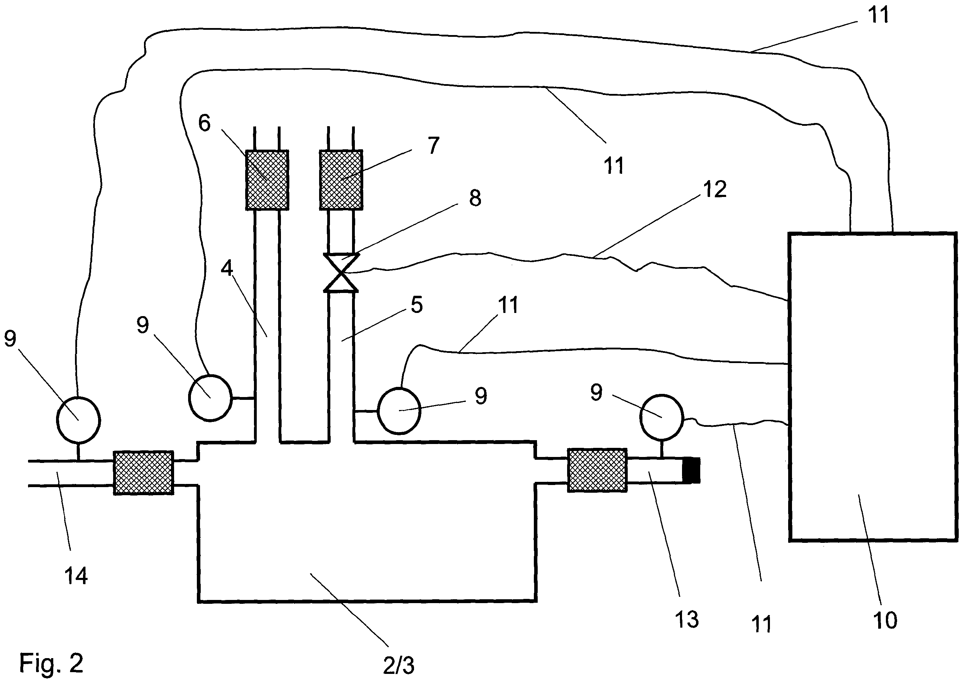

Entsprechend dem Ausführungsbeispiel von Figur 2 sind die Drucksensoren 9 auch in den Abluftleitungen 4, 5 und können in einer dritten Abluftleitung 13 angeordnet sein. Schließlich kann der Drucksensor 9 in einer Zuluftleitung 14 zum Einwegbehälter 3 angeordnet sein.

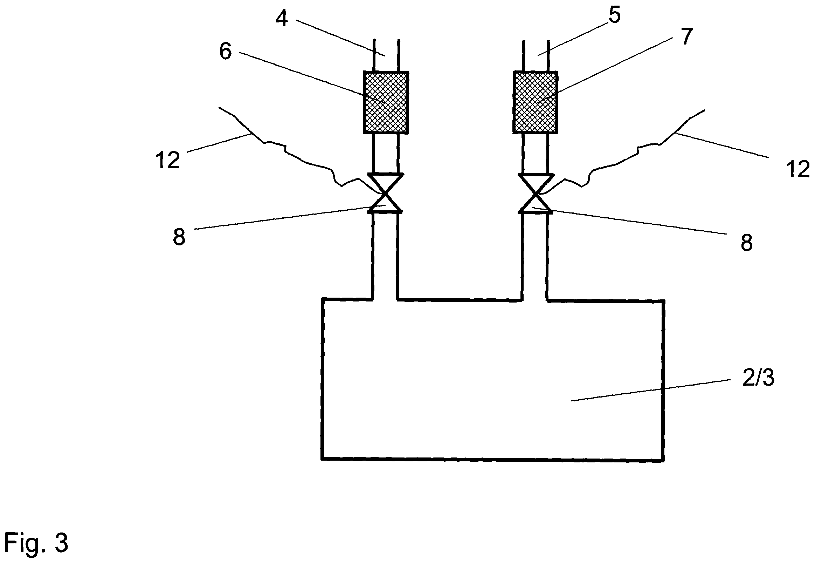

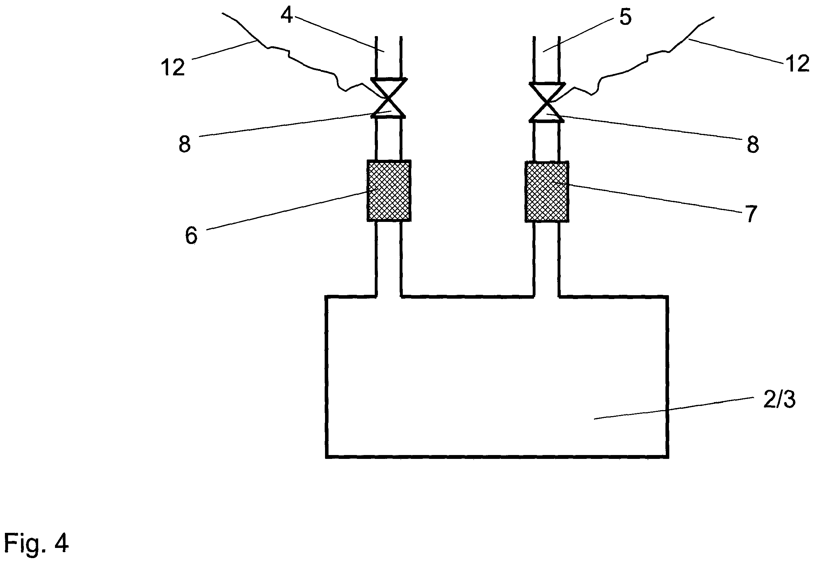

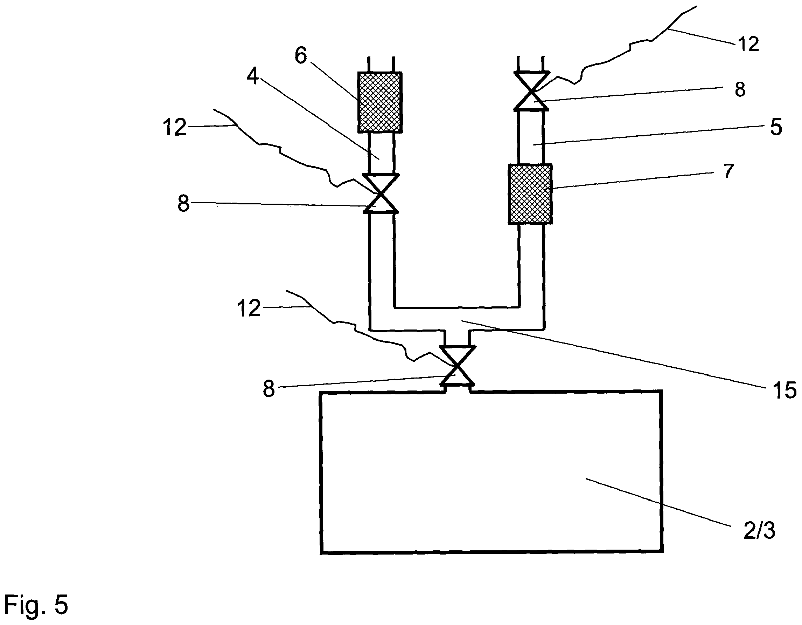

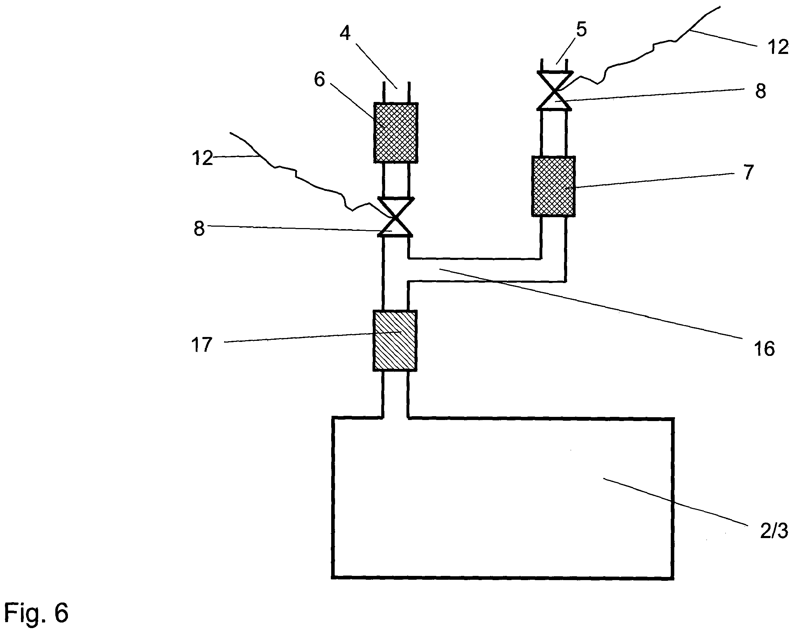

Entsprechend der Erfindung und gemäß der Figur 3 ist sowohl dem ersten Abluftfilter 6 als auch dem zweiten Abluftfilter 7 zum Einwegbehälter 3 hin jeweils ein Ventil 8 vorgelagert. Entsprechend dem Ausführungsbeispiel der Figur 4 können die Ventile 8 auch den Abluftfiltern 6, 7 vom Einwegbehälter 3 weggerichtet nachgelagert sein. Entsprechend den Figuren 5, 6 können die Abluftleitungen 4, 5 auch über ein Verteilerstück 15, 16 mit dem Einwegbehälter 3 in Verbindung stehen. Entsprechend dem Ausführungsbeispiel von Figur 5 ist dabei zwischen dem Verteilerstück 15 und dem Einwegbehälter 3 ein Ventil 8 angeordnet. Entsprechend dem Ausführungsbeispiel von Figur 6 ist zwischen dem Verteilerstück 16 und dem Einwegbehälter 3 ein Abluftkühler 17 angeordnet.

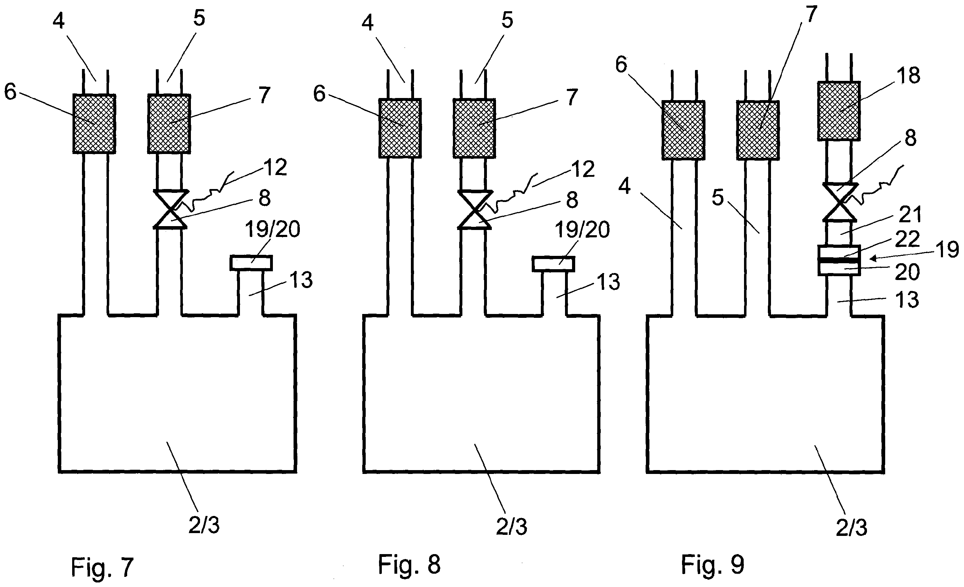

Die Figuren 7 bis 9 zeigen eine Prozessfolge, bei der ein nachträglicher unbenutzter dritter Abluftfilter 18 (oder weitere Abluftfilter) über einen zweiteiligen Sterilverbinder 19 und ein als Schlauchquetschventil ausgebildetes Ventil 8 angeschlossen wird. Figur 7 zeigt den Prozessstart mit geschlossenem Ventil 8 in der zweiten Abluftleitung 5, wobei die Abluft über die erste Abluftleitung 4 mit dem unverblockten ersten Abluftfilter 6 abgeleitet wird. Die dritte Abluftleitung 13 ist von einem ersten Teil 20 des zweiteiligen Sterilverbinders 19 verschlossen. Figur 8 zeigt den Prozess mit verblocktem ersten Abluftfilter 6 und geöffnetem Ventil 8 in der zweiten Abluftleitung 5. Damit ist die Abluft von der ersten Abluftleitung 4 auf die zweite Abluftleitung 5 umgeschaltet. Figur 9 zeigt den installierten dritten Abluftfilter 18, der mit einer Anschlussleitung 21 über ein zweites Teil 22 des Sterilverbinders 19 mit dem ersten Teil 20 verbunden wurde. Das Ventil 8, das als Schlauchquetschventil ausgebildet ist, wurde von der zweiten Abluftleitung 5 auf die Anschlussleitung 21 der dritten Abluftleitung 13 aufgesetzt und ist verschlossen. Die Abluftleitung 4 benötigt kein Ventil, da der erste Abluftfilter 6 verblockt ist und der zweite Abluftfilter 7 benötigt kein Ventil, da er in Funktion ist. Erst bei Verblockung des zweiten Abluftfilters 6 wird das Ventil 8 geöffnet.

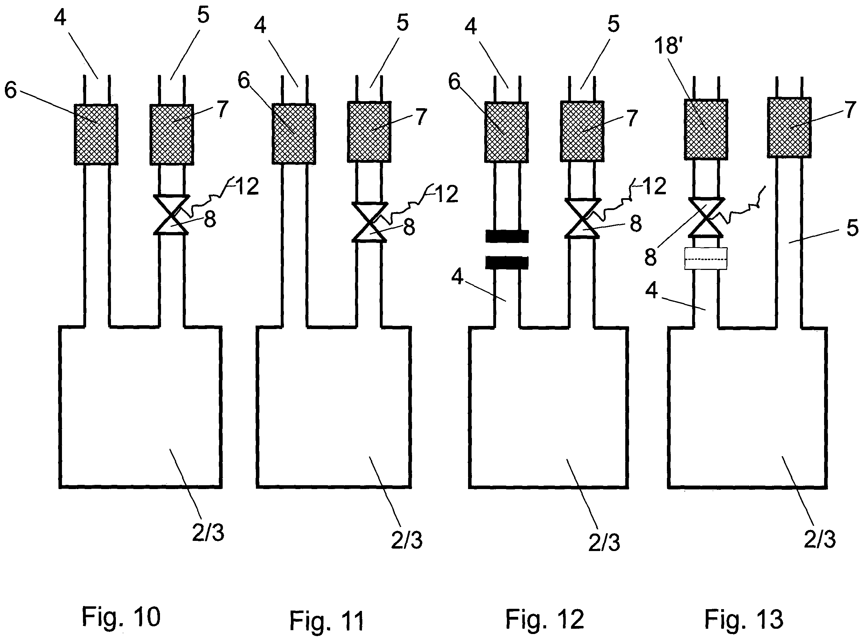

Die Figuren 10 bis 13 zeigen einen weiteren Prozess, bei dem nachträglich ein neuer unbenutzter Filter anstelle eines verblockten Filters angeschlossen wird. Figur 10 zeigt wieder den Prozessstart mit einem unverblockten ersten Abluftfilter 6 und einem noch unbenutzten zweiten Abluftfilter 7 mit vorgeschaltetem Ventil 8 in geschlossener Stellung. Das Ventil 8 im Ausführungsbeispiel der Figuren 10 bis 13 ist ebenfalls als Schlauchquetschventil ausgebildet. In Figur 11 ist der erste Abluftfilter 6 verblockt und der zweite Abluftfilter 7 bei geöffnetem Ventil 8 in Funktion. Figur 12 zeigt den ersten Abluftfilter 6, der durch "Sealing" (Versiegeln) steril von dem Einwegbehälter 3 abgetrennt ist. Das Ventil 8 in der Abluftleitung 5 ist weiterhin offen. Figur 13 zeigt einen dritten Abluftfilter 18', der mittels "Welding" (Verschweißen) steril mit der verbliebenen ersten Abluftleitung 4 verbunden wurde und dessen Ventil 8 wegen des noch nicht verblockten zweiten Abluftfilters 7 geschlossen ist und welcher nachfolgend durch Öffnen des Ventils 8 in Betrieb genommen werden kann, sofern der zweite Abluftfilter 7 im weiteren Prozessverlauf verblocken sollte.

Bei den Ausführungsbeispielen der Figuren 7 bis 13 können die Ventile 8 auch als Einwegventile (single-use-Ventile) ausgebildet sein.

### Bezugszeichenliste

- 1: System zur Abluftumschaltung
- 2: Bioreaktor
- 3: Einwegbehälter von 2
- 4: erste Abluftleitung
- 5: zweite Abluftleitung
- 6: erster Abluftfilter
- 7: zweiter Abluftfilter
- 8: Ventil
- 9: Sensor
- 10: Regel- und Steuereinheit
- 11: Sensorleitung
- 12: Steuerleitung
- 13: dritte Abluftleitung .
- 14: Zuluftleitung
- 15: Verteilerstück
- 16: Verteilerstück
- 17: Abluftkühler
- 18, 18': dritter Abluftfilter
- 19: Sterilverbinder
- 20: erster Teil des Sterilverbinders 19
- 21: Anschlussleitung
- 22: zweiter Teil des Sterilverbinders 19

## Patentansprüche

1. System (1) zur Abluftumschaltung eines Bioreaktors (2), dessen Einwegbehälter (3) über mindestens zwei Abluftleitungen (4, 5) mit Abluftfiltern (6, 7, 18, 18') in Verbindung steht,
wobei in mindestens einer der mindestens zwei Abluftleitungen (4, 5) ein Ventil (8) angeordnet ist,
**dadurch gekennzeichnet,**
**dass** über das Ventil (8) der zugehörige Abluftfilter (6, 7, 18, 18') freischaltbar ist,
**dass** in den Abluftleitungen (4, 5) Drucksensoren (9) vorgesehen sind,
**dass** eine Regel- und Steuereinheit (10) vorgesehen ist,
**dass** die Regel- und Steuereinheit (10) über Steuerleitungen (12) mit dem oder den Ventilen (8) verbunden ist und
**dass** die Regel- und Steuereinheit (10) über Sensorleitungen (11) mit den Drucksensoren (9) verbunden ist.

2. System zur Abluftumschaltung nach Anspruch 1,
**dadurch gekennzeichnet,**
**dass** die Abluftleitungen (4, 5) separat mit dem Einwegbehälter (3) verbunden sind.

3. System zur Abluftumschaltung nach Anspruch 1 oder 2,
**dadurch gekennzeichnet,**
**dass** die Abluftleitungen (4, 5) über ein Verteilerstück (15, 16) mit dem Einwegbehälter (3) verbunden sind.

4. System zur Abluftumschaltung nach einem der Ansprüche 1 bis 3,
**dadurch gekennzeichnet,**
**dass** mindestens eine weitere Abluftleitung (13) mit dem Einwegbehälter (3) in Verbindung steht, die an ihrem freien Ende von einem Teil (20) eines zweiteiligen Sterilverbinders (19) verschlossen ist.

5. System zur Abluftumschaltung nach einem der Ansprüche 1 bis 5, **dadurch gekennzeichnet, dass** die Ventile (8) den Abluftfiltern (6, 7) vor- oder nachgeschaltet angeordnet sind.

6. System zur Abluftumschaltung nach einem der Ansprüche 1 bis 6, **dadurch gekennzeichnet, dass** zwischen Einwegbehälter (3) und Abluftfilter (6, 7) ein Abluftkühler (17) angeordnet ist.

## Claims

1. A system (1) for switching over the exhaust of a bioreactor (2), the disposable container (3) of which is connected via at least two exhaust ducts (4, 5) with exhaust filters (6, 7, 18, 18'), wherein a valve (8) is arranged in at least one of the at least two exhaust ducts (4, 5), **characterized in that** the associated exhaust filter (6, 7, 18, 18') can be activated via the valve (8), that pressure sensors (9) are provided in the exhaust ducts (4, 5), that a control unit (10) is provided, that the control unit (10) is connected to the valve or valves (8) via control lines (12) and that the control unit (10) is connected to the pressure sensors (9) via sensor lines (11).

2. The system for switching over the exhaust according to Claim 1, **characterized in that** the exhaust ducts (4, 5) are separately connected to the disposable container (3).

3. The system for switching over the exhaust according to Claim 1 or 2, **characterized in that** the exhaust ducts (4, 5) are connected to the disposable container (3) via a distributor piece (15, 16).

4. The system for switching over the exhaust according to any of Claims 1 to 3, **characterized in that** at least one further exhaust duct (13) is connected to the disposable container (3), said further exhaust duct being sealed on its free end by a part (20) of a two-part sterile connector (19).

5. The system for switching over the exhaust according to any of Claims 1 to 5, **characterized in that** the valves (8) are arranged upstream or downstream of the exhaust filters (6, 7) .

6. The system for switching over the exhaust according to any of Claims 1 to 6, **characterized in that** an exhaust cooler (17) is arranged between the disposable container (3) and the exhaust filter (6, 7).

## Revendications

1. Système (1) destiné à la commutation d'une évacuation d'air d'un bioréacteur (2) dont le réservoir jetable (3) est en contact avec des filtres d'évacuation d'air (6, 7, 18, 18') via au moins deux conduites d'évacuation d'air (4, 5),
dans lequel une soupape (8) est agencée dans au moins l'une des au moins deux conduites d'évacuation d'air (4, 5),
**caractérisé en ce que**
le filtre d'évacuation d'air (6, 7, 18, 18') adéquat peut être librement commuté via la soupape (8),
des capteurs de pression (9) sont prévus dans les conduites d'évacuation d'air (4, 5),
une unité de régulation et de commande (10) est prévue,
l'unité de régulation et de commande (10) est reliée à la ou aux soupapes (8) via des conduites de commande (12) et
l'unité de régulation et de commande (10) est reliée aux capteurs de pression (9) via des câbles de capteur (11).

2. Système destiné à la commutation d'une évacuation d'air selon la revendication 1,
**caractérisé en ce que**
les conduites d'évacuation d'air (4, 5) sont reliées séparément au réservoir jetable (3).

3. Système destiné à la commutation d'une évacuation d'air selon la revendication 1 ou 2,
**caractérisé en ce que**
les conduites d'évacuation d'air (4, 5) sont reliées au réservoir jetable (3) via une pièce de répartiteur (15, 16).

4. Système destiné à la commutation d'une évacuation d'air selon l'une des revendications 1 à 3,
**caractérisé en ce que**
au moins une autre conduite d'évacuation d'air (13) est en contact avec le réservoir jetable (3), laquelle au niveau de son extrémité libre est fermée par une partie (20) d'un raccord stérile (19) bipartite.

5. Système destiné à la commutation d'une évacuation d'air selon l'une des revendications 1 à 5,
**caractérisé en ce que**
les soupapes (8) sont agencées en amont ou en aval des filtres d'évacuation d'air (6, 7).

6. Système destiné à la commutation d'une évacuation d'air selon l'une des revendications 1 à 6,
**caractérisé en ce que**
un refroidisseur d'évacuation d'air (17) est agencé entre le réservoir jetable (3) et le filtre d'évacuation d'air (6, 7).
